Europäisches Patentamt

**European Patent Office**

Office européen des brevets

(11) Veröffentlichungsnummer: **0 220 501**
**B1**

(12) EUROPÄISCHE PATENTSCHRIFT

(45) Veröffentlichungstag der Patentschrift:
**31.05.89**

(51) Int. Cl.⁴: **A61B 6/00**, B25J 9/00

(21) Anmeldenummer: **86113197.7**

(22) Anmeldetag: **25.09.86**

(54) Röntgendiagnostikanlage mit durch eine Steuervorrichtung verstellbaren Anlagenkomponenten.

(30) Priorität: **09.10.85 DE 3536079**

(43) Veröffentlichungstag der Anmeldung:
**06.05.87 Patentblatt 87/19**

(45) Bekanntmachung des Hinweises auf die Patenterteilung:
**31.05.89 Patentblatt 89/22**

(84) Benannte Vertragsstaaten:
**DE FR GB**

(56) Entgegenhaltungen:
**EP-A- 0 012 741**
**EP-A- 0 073 185**
**FR-A- 2 115 423**
**FR-A- 2 225 911**
**FR-A- 2 340 077**
**FR-A- 2 406 426**

**VDI-ZEITSCHRIFT, Band 125, Nr. 17, September 1983,
Seiten 647-652, Düsseldorf, DE; H. SCHÖLING et al.:
"Offline-Kollisionskontrolle bei Industrierobotern"**

(73) Patentinhaber: **Siemens Aktiengesellschaft Berlin und
München, Wittelsbacherplatz 2, D-8000 München 2(DE)**

(72) Erfinder: **Kresse, Heinz, Prof. Dr. rer. nat., Im
Pfarrgarten 8, D-8525 Uttenreuth(DE)**

## Beschreibung

Die Erfindung betrifft eine Röntgendiagnostikanlage mit einer verstellbaren Röntgenröhre, einem verstellbaren Bildaufnahmesystem und einer Patientenliege als Anlagenkomponenten und mit einer Steuervorrichtung für die Anlagenkomponenten. Eine Röntgendiagnostikanlage dieser Gattung ist aus FR-A 2 340 077 bekannt.

In der DE-OS 32 18 301 ist eine Röntgendiagnostikanlage mit einer Nachlaufsteuervorrichtung für die Verstellung der Röntgenröhre und des Strahlungsempfängers beschrieben. An einem an der Decke angebrachten Träger sind zwei Säulen verschiebbar angebracht, an deren freien Enden eine Röntgenröhre und ein Röntgenbildverstärker als Bildaufnahmesystem angeordnet sind. Die Länge der Säulen läßt sich variieren, so daß die Höhe der Rötgenröhre und des Röntgenbildverstärkers verändert werden kann. Ebenfalls können die Röntgenröhre und das Bildaufnahmesystem un die Säulenachse geschwenkt werden. Damit das Bildaufnahmesystem immer kauf die Röntgenröhre ausgerichtet ist, so daß das Röntgenstrahlenbündel die strahlenempfindliche Fläche trifft, ist eine Nachlaufsteuervorrichtung vorgesehen, die eine elektrische Koppelung beider Komponenten bewirkt. Durch die starren Säulen lassen sich ohne aufwendige mechanische Konstruktionsteile nicht alle gewünschten Aufnahmepositionen erreichen, so daß eine derartige Röntgendiagnostikanlage nicht universell eingesetzt werden kann.

Die Erfindung geht von der Aufgabe aus, eine Röntgendia gnostikanlage der eingangs genannten Art derart auszubilden, daß eine freie Beweglichkeit der Anlagenkomponenten und somit ein universeller Einsatz gewährleistet sind.

Die Aufgabe wird erfindungsgemäß dadurch gelöst, daß wenigstens zwei der Anlagenkomponenten individuell dreidimensional mit Hilfe motorisch betätigbarer Stellmittel bewegbar gehalten sind und daß als Steuervorrichtung ein zentraler Rechner mit den Stellmitteln zur Vorgabe der Position der Komponenten verbunden ist. Dadurch lassen sich nahezu alle Positionen der Anlagenkomponenten beliebig wählen, während der Rechner die Ausrichtung der Anlagenkomponenten zueinander bewirkt, so daß dadurch die Anlagenkomponenten verbindende konstruktive Elemente kentfallen.

Die freie Zugänglichkeit der Untersuchungspersonen während der Untersuchung wird erreicht, wenn die Stellmittel nach Art von Roboterarmen aufgebaut sind, die einen an einem Haltepunkt dreh- und schwenkbar angebrachten ersten Hebel aufweisen, der an seinem freien Ende mit einem zweiten Hebel schwenkbar verbunden ist, der mit der jeweiligen Anlagenkomponente dreh- und schwenkbar befestigt ist. Wegen der kleinen zu bewegenden Masse der Roboterarme lassen sich beispielsweise Parkstellungen schneller erreichen, in denen die Tragarme einen geringeren Platzbedarf benötigen. Verschiedene, vor der Untersuchung ermittelte Positionen lassen sich leicht abrufen, wenn der Rechner Speichermittel für verschiedene einprogrammierbare feste Positionen aufweist.

Eine knotinuierliche Bewegung der Anlagenkomponenten nach einer Funktion wird erreicht, wenn der Rechner mehrere Programmspeicher für unterschiedliche Programmabläufe aufweist. Mit der Röntgendiagnostikanlage lassen sich Schichtaufnahmen durchführen, wenn die Programmspeicher derart ausgebildet sind, daß die Steuervorrichtung die Röntgenröhre und das Bildaufnahmesystem gegenläufig zueinander in zwei zueinander parallelen Ebenen bewegt. Einfache Computertomographieaufnahmen lassen sich durchführen, wenn die Programmspeicher derart ausgebildet sind, daß die Röntgenröhre und das Bildaufnahmesystem auf einer Kreisbahn um ihren gemeinsamen Mittelpunkt bewegt werden, wobei die Röntgenröhre mit ·einer Strahlenblende versehen ist, so daß sie ein fächerförmiges Röntgenstrahlenbündel aussendet. Drehmuldenartige Bewegungen der Patientenliege lassen sich erreichen, wenn die Programmspeicher entsprechend ausgebildet sind. Durch ein entsprechendes Programm können allgemein die erforderlichen Bewegungen, z.B. Hin- und Herbewegungen, Auf- und Abbewegungen, erreicht werden.

Der Arbeitsbereich läßt sich erweitern, wenn die Stellmittel in Schienen verfahrbar geführt sind. Als Sicherheitsvorrichtung für die frei beweglichen Anlagenkomponenten läßt sich vorteilhaft wenigstens eine Fernsehkamera auf die Röntgendiagnostikanlage ausrichten, die mit dem Rechner derart verbunden ist, daß sie diejenigen Position sperrt, in denen sich von der Fernsehkamera erkannte Gegenstände oder Menschen befinden.

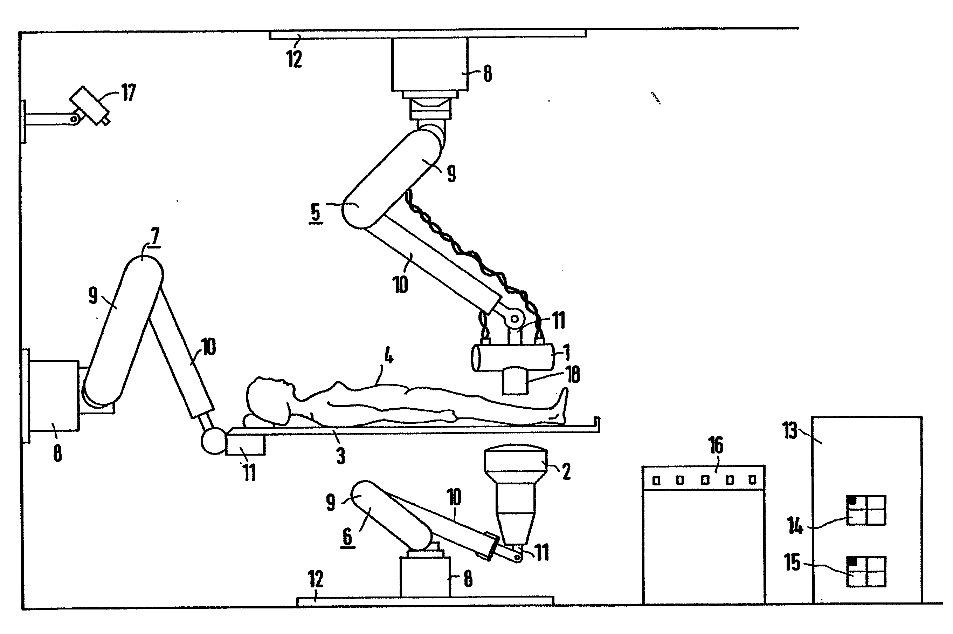

Die Erfindung ist nachfolgend anhand eines in der Zeichnung dargestellten Ausfürungsbeispieles näher erläutert.

In der Figur ist eine erfindungsgemäße Röntgendiagnostikanlage mit einer Röntgenröhre 1, einem Röntgenbildverstärker 2 als Bildaufnahmesystem und einer Patientenliege 3 mit einem Patienten 4 dargestellt. Diese Anlagenkomponenten 1 bis 3 sind durch Tragarme 5 bis 7 als Stellmittel gehalten, die nach Art von Roboterarmen aufgebaut sind.

Sie weisen einen entweder mit wand, Decke oder Boden des Untersuchungsraumes verbundenen Sockel 8 auf, an dem ein erster hebel 9 motorisch dreh- und schwenkbar angebracht ist. Mit seinem freien Ende ist ein zweiter Hebel 10 verbunden, der durch einen nicht dargestellten Motor geschwenkt werden kann. Der zweite Hebel 10 ist über ein ebenfalls motorisch betätigbares Gelenk mit einer Halterung 11 für die Anlagenkomponenten 1 bis 3 verbunden, so daß diese dreh- und schwenkbar sind. Zur Erweiterung des Einsatzbereiches der Anlagenkomponenten 1 bis 3 können die Sockel 8 auf Schienen 12 motorisch verschiebbar montiert sein, wie dies für die Tragarme 5 und 6 der Röntgenröhre 1 und des Röntgenbildverstärkers 2 dargestellt ist.

Die Motoren der Tragarme 5 bis 7 sind mit einem zentralen Rechner 13 als Steuervorrichtung verbunden, der Speichermittel 14, 15 für verschiedene feste Positionen und unterschiedliche Programmabläufe für Knotinuierliche Bewegungen der Anlagenkomponenten aufweist. Von einem mit dem Rechner 13 verbundenen Steuerpult 16 lassen sich die verschiedenen Programme, Positionen oder Funktionen abrufen. So lassen sich beispielswiese in dem Speicher 14 verschiedene, vor der Aufnahme mit Lichtviser ermittelte Positionen einspeichern, die später während der Untersuchung auf Tastendruck abgerufen werden können. Auch lassen sich auf Knopfdruck die Abstände der Anlagenkomponenten 1 bis 3 zueinander und somit der Vergrößerungsfaktor und die Bildschärfe aus dem Speicher 14 abrufen. Anstelle des Steuerpultes 16 kann der rechner 13 zur Bedienung der Anlagenkomponenten 1 bis 3 auch mit einer nicht dargestellten Spracheingabe versehen sein.

In dem programmspeicher 15 können mehrere verschiedene Bewegungsabläufe der Anlagenkomponentem 1 bis 3 abgespeichert sein. So kann die für die Schichttechnik gegenläufige Bewegung der Röntgenröhre 1 und des Röntgenbildverstärkers 2 in zwei zueinander parallelen Ebenen als lineare, kreisförmige oder beliebige Verwischung in dem Programmspeicher 15 enthalten sein. Auch lassen sich computertomographische Aufnahmen einer Schicht erstellen, wenn der Programmspeicher 15 ein Programm enthält, so daß die Steuervorrichtung die Tragarme 5 und 6 derart steuart, daß sich die Röntgenröhre 1 und der Röntgenbildverstärker 2 kreisförmig um die Längsachse des Patienten 4 als gemeinsamer Mittelpunkt bewegen. Hierzu wird die Primärstrahlenblende 18 der Röntgenröhre 1 derart eingestellt, daß sie ein fächerförmiges Strahlenbündel aussendet, das vom Röntgenbildverstärker 2 oder einem anderen geeigneten Strahlenempfänger empfangen wird. Die Ausgangssignale beispielsweise des Röntgenbildverstärkers 2 werden dem Rechner 13 zugeführt, der aus den zu verschiedenen Positionen gehörenden Signalen ein CT-Bild berechnet.

Zur Steuerung des Tragarmes 7 der Patientenliege 3 durch in einem weiteren Speicherplatz des Programmspeichers 15 enthaltene Programme können drehmuldenartige Bewegungen, gesteuerte Tieflagen oder eine Umlagerung des Patienten vom Transportgerät zum Röntgengerät bewirkt werden. Eine Verschiebung des Untersuchungsgebietes in Richtung der Längsachse des Patienten 4 kann entweder durch Bewegung des Tragarmes 7 der Patientenliege 3 oder durch gekoppelte Bewegung der Tragarme 5 und 6 der Röntgenröhre 1 und des Röntgenbildverstärkers 2 oder durch beide Maßnahmen zugleich bewirkt werden. Der Tragarm 7 der Patientenliege 3 kann aber auch einen normalen, beispielsweise säulenartigen Aufbau aufweisen, da die freie Beweglichkeit de Röntgenröhre 1 und des Röntgenbildverstärkers 2 in vielen Fällen ausreicht.

Zur Verhinderung der Gefährdung von Patienten, Personal und der Röntgendiagnostikanlage durch sich bewegende Geräteteile können diese mit optischen, taktilen oder Ultraschallsensoren in bekannter Weise ausgestattet sein. Es können aber auch eine oder mehrere mit dem Rechner 13 verbundene Fernsehkameras 17 vorgesehen sein, die die Umrisse von beteiligten Personen und Gegenständen erfassen und nach Bildverarbeitung diese Raumpositionen, in denen sich die Personen und Gegenstände befinden, für die sich bewegenden Geräteteile sperren.

## Patentansprüche

1. Röntgendiagnostikanlage mit einer verstellbaren Röntgenröhre (1), einem verstellbaren Bildaufnahmesystem (2) und einer Patientenliege (3) als Anlagenkomponenten (1 bis 3) und mit einer Steuervorrichtung (13) für die Anlagenkomponenten (1 bis 3), **dadurch gekennzeichnet,** daß wenigstens zwei der Anlagenkomponenten (1 bis 3) individuell dreidimensional mit Hilfe motorisch betätigbarer Stellmittel (5 bis 7) bewegbar gehalten sind und daß als Steuervorrichtung ein zentraler Rechner (13) mit den Stellmitteln (5 bis 7) zur Vorgabe der Position der Anlagenkomponenten (1 bis 3) verbunden ist.

2. Röntgendiagnostikanlage nach Anspruch 1, **dadurch gekennzeichnet,** daß die Stellmittel (5 bis 7) nach Art von Roboterarmen aufgebaut sind, die einen an einem Haltepunkt (8) dreh- und schwenkbar angebrachten ersten Hebel (9) aufweisen, der an seinem freien Ende mit einem zweiten Hebel (10) schwenkbar verbunden ist, der mit der jeweiligen Anlagenkomponente (1 bis 3) dreh- und schwenkbar befestigt ist.

3. Röntgendiagnostikanlage nach Anspruch 1, **dadurch gekennzeichnet,** daß der Rechner (13) Speichermittel (14) für verschiedene einprogrammierbare feste Positionen aufweist.

4. Röntgendiagnostikanlage nach Anspruch 1, **dadurch gekennzeichnet,** daß der Rechner (13) mehrere Programmspeicher (15) für unterschiedliche Programmabläufe aufweist.

5. Röntgendiagnostikanlage nach Anspruch 4, **da durch gekennzeichnet,** daß die Programmspeicher (15) derart ausgebildet sind, daß die Steuervorrichtung (13) die Röntgenröhre (1) und das Bildaufnahmesystem (2) gegenläufig zueinander in zwei zueinander parallelen Ebenen bewegt.

6. Röntgendiagnostikanlage nach Anspruch 4, **dadurch gekennzeichnet,** daß die Programmspeicher (15) derart ausgebildet sind, daß die Röntgenröhre (1) und das Bildaufnahmesystem (2) auf einer Kreisbahn um ihren gemeinsamen Mittelpunkt bewegt werden, wobei die Röntgenröhre (1) mit einer Strahlenblende (18) versehen ist, daß sie ein fächerförmiges Röntgenstrahlenbündel aussendet.

7. Röntgendiagnostikanlage nach Anspruch 4, **dadurch gekennzeichnet,** daß die Programmspeicher (15) derart ausgebildet sind, so daß unterschiedliche Bewegungen der Patientenliege (3) möglich sind.

8. Röntgendiagnostikanlage nach Anspruch 1, **dadurch gekennzeichnet,** daß die Stellmittel (5 bis 7) in Schienen (12) verfahrbar geführt sind.

9. Röntgendiagnostikanlage nach Anspruch 1 mit Sicherheitsvorrichtungen, **dadurch gekennzeichnet,** daß wenigstens eine Fernsehkamera (17) auf die Röntgendiagnostikanlage ausgerichtet ist, die mit dem Rechner (13) derart verbunden ist, daß sie diejenigen Positionen sperrt, in denen sich von der Fersehkamera (17) erkannte Gegenstände oder Menschen befinden.

## Claims

1. A diagnostic X-ray apparatus having an adjustable X-ray tube (1), an adjustable image pick-up system (2) and a patient couch (3) and having a control device (13) for the components (1–3) of the apparatus, characterised in that at least two of the components (1–3) of the apparatus are mounted to be individually movable in three dimensions by means of motor-operable adjusting means (5 to 7), and that as the control device a central computer (13) is connected to the adjusting means (5 to 7) to set the positions of the components (1 to 3) of the apparatus.

2. A diagnostic X-ray apparatus according to claim 1, characterised in that the adjusting means (5 to 7) are constructed as robot arms which have a first lever (9) rotatably and pivotably attached to a holding point (8) and pivotably connected at its free end to a second lever (10) which is fixed rotatably and pivotably to the respective component (1 to 3) of the apparatus.

3. A diagnostic X-ray apparatus according to claim 1, characterised in that the computer (13) has storage means (14) for different programmable fixed positions.

4. A diagnostic X-ray apparatus according to claim 1, characterised in that the computer (13) has a plurality of programme stores (15) for different programme routines.

5. A diagnostic X-ray apparatus according to claim 4, characterised in that the programme stores (15) are formed so that the control device (13) moves the X-ray tube (1) and the image pick-up system in opposite directions to one another in two planes parallel to one another.

6. A diagnostic X-ray apparatus according to claim 4, characterised in that the programme stores (15) are formed so that the X-ray tube (1) and the image pick-up system (2) are moved on a circular path about their common centre point, the X-ray tube (1) being provided with a radiation diaphragm (18), so that it emits a fan-shaped bundle of rays.

7. A diagnostic X-ray apparatus according to claim 4, characterised in that the programme stores (15) are instructed so that different movements of the patient couch (3) are possible.

8. A diagnostic X-ray apparatus according to claim 1, characterised in that the adjusting means (5 to 7) are movably guided in rails (12).

9. A diagnostic X-ray apparatus according to claim 1 having safety devices, characterised in that at least one television camera (17) is aligned with the diagnostic X-ray apparatus, and is connected to the computer (13) in such a way that it blocks those positions in which there are objects or people recognised by the television camera (17).

## Revendications

1. Installation de radiodiagnostic comportant un tube à rayons X (1) réglable, un système réglable (2) de prise de vues et une couchette pour patient (3), en tant que composants (1 à 3) de l'installation, et un dispositif de commande (13) pour les composants (1 à 3) de l'installation, caractérisée par le fait qu'au moins deux des composants (1 à 3) de l'installation sont maintenus de manière à être déplaçables individuellement, dans trois dimensions, à l'aide de moyens de réglage (5 à 7) pouvant être actionnés par des moteurs, et qu'un ordinateur central (13) est relié, en tant que dispositif de commande, aux moyens de réglage (5 à 7) de manière à prédéterminer la position des composants (1 à 3) de l'installation.

2. Installation de radiodiagnostic suivant la revendication 1, caractérisée par le fait que les moyens de réglage (5 à 7) sont réalisés à la manière de bras de robots, qui possèdent un premier levier (9) monté de manière à pouvoir tourner et basculer autour d'un point de maintien (8) et relié, de manière à pouvoir basculer, au niveau de son extrémité libre, à un second levier (10) fixé de manière à pouvoir pivoter et basculer aux composants respectifs (1 à 3) de l'installation.

3. Installation de radiodiagnostic suivant la revendication 1, caractérisée par le fait que l'ordinateur (13) comporte des moyens de mémoire (14) pour différentes positions fixes programmables.

4. Installation de radiodiagnostic suivant la revendication 1, caractérisée par le fait que l'ordinateur (13) comporte plusieurs mémoires de programmes (15) pour différentes exécutions de programmes.

5. Installation de radiodiagnostic suivant la revendication 4, caractérisée par le fait que la mémoire de programmes (15) est agencée de telle sorte que le dispositif de commande (13) déplace le tube à rayons X (1) et le système (2) de prise de vues en des sens opposés l'un à l'autre, dans deux plans parallèles.

6. Installation de radiodiagnostic suivant la revendication 4, caractérisée par le fait que la mémoire de programmes (15) est agencée de manière que le tube à rayon X (1) et le système (2) de prise de vues sont déplacés sur une trajectoire circulaire autor de leur centre commun, le tube à rayons X (1) étant pourvu d'un diaphragme de rayonnement (18) de sorte qu'il émet un faisceau de rayons X en forme d'éventail.

7. Installation de radiodiagnostic suivant la revendication 4, caractérisée par le fait que la mémoire de programmes (15) est agencée de manière à permettre différents déplacements de la couchette pour patient (3).

8. Installation de radiodiagnostic suivant la revendication 1, caractérisée par le fait que les moyens de réglage (5 à 7) sont guidés de manière à être déplaçables dans des rails (12).

9. Installation de radiodiagnostic suivant la revendication 1, comportant des dispositifs de sécurité, caractérisée par le fait qu'au moins une caméra de télévision (17) est alignée sur l'installation de radiodiagnostic, qui est reliée à l'ordinateur (13) de manière à se bloquer dans des positions, dans lesquelles sont situés des objets ou des personnes, identifiés par la caméra de télévision (17).

EP 0 220 501 B1